# EUROPEAN PATENT SPECIFICATION

(11) **EP 4 640 711 B1**
(45) Date of publication and mention of the grant of the patent: **28.10.2026**
(21) Application number: 23909713.2
(22) Date of filing: 09.11.2023
(51) Int. Cl.: C07K 16/28, A61K 39/00, A61K 39/395, A61P 35/00, C12N 5/10, C12N 15/13, C12N 15/85, G01N 33/575

(54) **HIGH-AFFINITY TIGIT ANTIBODY AND USE THEREOF**
HOCHAFFINER TIGIT-ANTIKÖRPER UND VERWENDUNG DAVON
ANTICORPS ANTI-TIGIT À HAUTE AFFINITÉ ET SON UTILISATION

(30) Priority: 30.12.2022 CN 202211720208
(43) Date of publication of application: 29.10.2025
(73) Proprietor: Hefei TG Immunopharma Co., Ltd., Hefei, Anhui 230001 (CN)
(72) Inventor: CAO, Guoshuai, Hefei, Anhui 230001 (CN); CHENG, Ying, Hefei, Anhui 230001 (CN); LI, Yangyang, Hefei, Anhui 230001 (CN); WU, Yuwei, Hefei, Anhui 230001 (CN)
(74) Representative: Gill Jennings & Every LLP
(86) International application number: PCT/CN2023/130805
(87) International publication number: WO 2024/139781

(56) References cited:
- WO-A1-2016/106302
- WO-A1-2022/257279
- WO-A2-2017/053748
- CN-A- 102 057 272
- CN-A- 109 384 846
- CN-A- 109 384 846
- CN-A- 110 997 720
- CN-A- 112 679 610
- CN-A- 114 181 310
- CN-A- 116 143 923
- US-A1- 2020 331 999
- US-A1- 2021 087 268

## Description

### FIELD

The present disclosure relates to the field of antibodies, and in particular, to a high-affinity TIGIT antibody and a use thereof. More particularly, the present disclosure relates to an antibody or an antigen-binding fragment thereof, a nucleic acid molecule, an expression vector, a method for preparing the antibody or the antigen-binding fragment thereof, a recombinant cell, a composition and a use thereof, a medicament and a use thereof for treating a malignant tumor.

### BACKGROUND

Malignant tumors are a serious threat to human life and health. Currently available treatments for malignant tumors, including surgical resection, radiotherapy, chemotherapy, small molecule targeted therapy, immune checkpoint therapy, and cell and gene therapy, can only play a limited role in some patients with malignant tumors, and malignant tumors remain a problem plaguing human life and health.

In recent years, immunotherapy has made remarkable progress in the field of tumor treatment, especially immune checkpoint blockade therapy represented by anti-CTLA-4 and anti-PD-1 or PD-L1 antibodies. By blocking the binding of inhibitory receptors on the surface of T cells to their ligands, blocking the transmission of inhibitory signals, correcting the immunosuppression mediated by the immunosuppressive microenvironment, and restoring the anti-tumor ability of T cells in the tumor microenvironment, it has achieved a high response rate in the treatment of various metastatic advanced malignant tumors (including metastatic melanoma, non-small cell lung cancer, and renal cancer). However, it cannot be ignored that even the currently approved PD-1/L1 therapy with the widest indications has an overall response rate of only 30%. There are still many more patients who cannot benefit from it.

However, not all patients with malignant tumors respond effectively to PD-1, PD-L1 or CTLA-4 blockade therapy. Only 10%-30% of patients exhibit a long-lasting response to PD-1 or PD-L1 antibody treatment, and the majority of patients fail to respond. On the one hand, due to the significant heterogeneity in the expression of receptors and ligands of immune checkpoints against tumors and tumor-infiltrating lymphocytes, a single type of immune checkpoint therapy cannot be applied to all patients, and the majority of patients cannot benefit from it; on the other hand, some patients who have received immune checkpoint therapy may experience tumor recurrence and develop tolerance to this immune checkpoint therapy, while continued administration still fails to produce efficacy,which is also known as secondary drug resistance. For these two reasons, it is necessary to develop immune checkpoint antibodies against more targets.

T-cell immunoglobulin and ITIM (immunoreceptor tyrosine-based inhibitory motif) domain protein (TIGIT), an important immune checkpoint, is predominantly expressed on the surface of natural killer (NK) cells, activated CD8+ T and CD4+ T cells, regulatory T cells (Tregs) and follicular helper T cells (Tfh). The ligands recognized by TIGIT, CD155 and CD112, are predominantly expressed on monocytes, macrophages, dendritic cells (DCs), T cells, B cells and many non-hematopoietic cells (including tumor cells of various histological types). The binding affinity of TIGIT to the ligand PVR (polivirus receptor, CD155) is significantly higher than that of its competing receptors CD226 and CD96. TIGIT binds to its ligand and transmits inhibitory signals through the intracellular ITIM motif, thereby inhibiting the functions of T cells and NK cells.

Studies have shown that the expression of TIGIT is associated with the development of various malignant tumors (e.g., non-small cell lung cancer, melanoma, head and neck squamous cell carcinoma, colorectal cancer, glioblastoma, gastric cancer, liver cancer, multiple myeloma, acute myeloid leukemia and follicular lymphoma). It has been reported that TIGIT molecules are highly expressed on the surface of tumor-infiltrating T cells in patients with lung cancer, and there is a significant positive correlation between the expression and PD-1 expression. The expression of TIGIT on the surface of tumor-infiltrating NK cells in patients with colon cancer is significantly higher than that on peripheral blood NK cells. In various mouse tumor models, TIGIT blockade with antibodies can inhibit tumor growth and metastasis, while concurrent blockade of TIGIT and PD-1 can effectively treat tumors. Therefore, the development of therapeutic TIGIT antibodies is particularly necessary.

CN109384846A discloses antagonistic human monoclonal anti-huTIGIT antibodies, followed by characterization and humanization of the resulting antibody candidate clones. WO2016/106302A1 discloses the production of antagonistic human monoclonal anti-TIGIT antibodies by immunizing transgenic mice, that produce human antibodies, with the huTIGIT protein. WO2017/053748A2 discloses antagonistic human monoclonal anti-huTIGIT antibodies. WO2022/257279A1 discloses antagonistic human monoclonal anti-TIGIT antibodies. CN114181310A discloses the production of antagonistic human monoclonal anti-TIGIT antibodies by immunizing mice with huTIGIT, followed by humanization of the resulting antibody clones.

### SUMMARY

The present disclosure is intended to address at least one of the technical problems existing in the related art to some extent.

To inhibit tumor growth and metastasis, as well as to address secondary drug resistance that arises, the present disclosure provides a high-affinity TIGIT antibody and its use in the treatment of a malignant tumor.

By blocking the binding of TIGIT to the ligand CD155, the antibody provided by the present disclosure relieves TIGIT-mediated immunosuppression and promotes the anti-tumor function of immune cells.

Therefore, to achieve the above objectives, in a first aspect, the present disclosure provides an antibody or an antigen-binding fragment thereof capable of specifically recognizing T cell immunoreceptor with Ig and ITIM domains (TIGIT), comprising a heavy chain variable region and a light chain variable region, wherein the amino acid sequence of the heavy chain variable region is as set forth in SEQ ID NO: 7, and the amino acid sequence of the light chain variable region is as set forth in SEQ ID NO: 8. Further embodiments of the antibodies of the invention are set out in the claims.

QVQLQESGPGLVKPSQTLSLTCTVTGSSITSDYAWNWIRQFPGKKLEWMG
YITYSGRTTYNPSLKSRITISRDTSKNQFSLKLSSVTAADTATYYCARWGLLRRYFDYW

GQGTLLTVSS (SEQ ID NO: 7),

DIVMTQSPSSMSTSVGDRVTITCRASQDVFNQVAWYQQKPGKSPKLLIYSA
SFRYTGVPDRFSGSGSGTDFTFTISSVQAEDFATYYCQQHYSTPLTFGAGTKLELK
(SEQ ID NO: 8).

According to an embodiment of the present disclosure, the antibody is a humanized antibody.

According to an embodiment of the present disclosure, the antibody or the antigen-binding fragment thereof includes a heavy chain framework region sequence and a light chain framework region sequence, wherein at least a portion of at least one of the heavy chain framework region sequence and the light chain framework region sequence is from at least one of a murine antibody, a human antibody, a primate antibody, or a mutant thereof.

According to an embodiment of the present disclosure, the antibody or the antigen-binding fragment thereof includes at least one of a heavy chain constant region and a light chain constant region, wherein at least a portion of at least one of the heavy chain constant region and the light chain constant region is from at least one of a human antibody, a primate antibody, a murine antibody, or a mutant thereof.

According to an embodiment of the present disclosure, both the light chain constant region and the heavy chain constant region are from a murine IgG1 antibody, IgG2a antibody, or a mutant thereof, or a human IgG1 antibody, IgG2 antibody, IgG3 antibody, IgG4 antibody, or a mutant thereof. According to an embodiment of the present disclosure, the antibody or the antigen-binding fragment thereof is at least one of a single-chain antibody, a multimeric antibody, a CDR-grafted antibody, a Fab antibody, and an Fv antibody.

In a second aspect, the present disclosure provides a nucleic acid molecule. According to an embodiment of the present disclosure, the nucleic acid molecule encodes the antibody or the antigen-binding fragment thereof according to the first aspect of the present disclosure.

The antibody or the antigen-binding fragment thereof encoded by the nucleic acid molecule according to an embodiment of the present disclosure exhibits stronger specificity, a longer half-life and higher potency, enabling effective treatment or prevention of malignant tumors at lower dosages with reduced toxic side effects and improved safety.

In a third aspect, the present disclosure provides an expression vector. According to an embodiment of the present disclosure, the expression vector carries the nucleic acid molecule according to the second aspect of the present disclosure.

The expression vector provided by an embodiment of the present disclosure can efficiently express the above antibody or the antigen-binding fragment thereof in suitable host cells. The antibody or the antigen-binding fragment thereof, designed by the present disclosure, exhibits stronger specificity and higher safety.

In a fourth aspect, the present disclosure provides a method for preparing the above antibody or the antigen-binding fragment thereof. According to an embodiment of the present disclosure, the method includes:
introducing the expression vector according to the third aspect of the present disclosure into a cell; and
culturing the cell under conditions suitable for protein expression and secretion to obtain the antibody or the antigen-binding fragment thereof.

The inventors have found that the antibody or the antigen-binding fragment thereof prepared according to an embodiment of the present disclosure enables more efficient production of a high-purity antibody or an antigen-binding fragment thereof, with simplified procedures and reduced costs.

According to an embodiment of the present disclosure, the cell is a eukaryotic cell.

In a fifth aspect, the present disclosure provides a recombinant cell. According to an embodiment of the present disclosure, the recombinant cell expresses the antibody or the antigen-binding fragment thereof according to the first aspect of the present disclosure, or carries the nucleic acid molecule according to the second aspect of the present disclosure or the expression vector according to the third aspect of the present disclosure.

According to some specific embodiments of the present disclosure, the recombinant cell can efficiently and massively express the antibody or the antigen-binding fragment thereof under suitable conditions. The antibody or the antigen-binding fragment thereof exhibits stronger specificity, a longer half-life and higher potency, enabling delivery of the antibody drug to target cells at lower dosages to achieve effective treatment of diseases or prevention of cancers with reduced toxic side effects and improved safety.

In a sixth aspect, the present disclosure provides a composition. According to an embodiment of the present disclosure, the composition includes: the antibody or the antigen-binding fragment thereof according to the first aspect of the present disclosure, the nucleic acid molecule according to the second aspect of the present disclosure, the expression vector according to the third aspect of the present disclosure, or the recombinant cell according to the fifth aspect of the present disclosure.

According to an embodiment of the present disclosure, the composition of the present disclosure can achieve a comparable therapeutic effect to the antibody or the antigen-binding fragment thereof, the nucleic acid molecule, the expression vector, or the recombinant cell when introduced into an organism.

In a seventh aspect, the present disclosure provides use of the composition in the preparation of a medicament. According to an embodiment of the present disclosure, the medicament is for use in the treatment or prevention of a malignant tumor.

According to an embodiment of the present disclosure, the antibody or the antigen-binding fragment thereof can be used for the preparation of a medicament. The prepared medicament exhibits comparable efficacy to the antibody or the antigen-binding fragment thereof and can be used for the treatment or prevention of diseases such as a malignant tumor.

According to an embodiment of the present disclosure, the malignant tumor includes at least one of: lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, or head and neck cancer.

In an eighth aspect, the present disclosure provides a medicament. According to an embodiment of the present disclosure, the medicament includes: the antibody or the antigen-binding fragment thereof according to the first aspect of the present disclosure, the nucleic acid molecule according to the second aspect of the present disclosure, the expression vector according to the third aspect of the present disclosure, the recombinant cell according to the fifth aspect of the present disclosure, or the composition according to the sixth aspect of the present disclosure, and the medicament is for use in the treatment of a malignant tumor.

The inventors have found that a medicament including the antibody or the antigen-binding fragment thereof, the nucleic acid molecule, the expression vector, the recombinant cell, or the composition exhibits superior efficacy for the treatment and prognosis of a malignant tumor.

According to an embodiment of the present disclosure, the malignant tumor includes at least one of: lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, or head and neck cancer.

In a ninth aspect, the present disclosure provides the use of the above antibody or the antigen-binding fragment thereof in the preparation of a kit. According to an embodiment of the present disclosure, the kit is for use in the detection of TIGIT.

According to an embodiment of the present disclosure, the kit enables more efficient and precise detection of TIGIT, thereby saving time and exploration costs for clinical treatment.

In a tenth aspect, the present disclosure provides a kit. According to an embodiment of the present disclosure, the kit includes the antibody or the antigen-binding fragment thereof according to the first aspect of the present disclosure.

The inventors have found that a kit prepared based on the antibody or the antigen-binding fragment thereof can be used in scientific research, saving time and costs consumed in the analysis of a malignant tumor.

### BRIEF DESCRIPTION OF DRAWINGS

The above and/or additional aspects and advantages of the present disclosure will be obvious and readily appreciated from the following description of examples, taken in conjunction with the accompanying drawings, in which:
FIG. 1 shows the ELISA results of TIGIT antibody H401LV2 (H401LV2-hIgG1, H401LV2-hIgG1LALA, H401LV2-hIgG4S228P) binding to human and monkey TIGIT according to Example 3 of the present disclosure;
FIG. 2 shows the ELISA results of TIGIT antibody H401LV2 (H401LV2-hIgG1, H401LV2-hIgG1LALA, H401LV2-hIgG4S228P) blocking the binding of TIGIT to CD155 according to Example 4 of the present disclosure;
FIG. 3 shows the results of the TIGIT antibody H401LV2 (H401LV2-hIgG1, H401LV2-hIgG1LALA, H401LV2-hIgG4S228P) binding to 293T-TIGIT cells according to Example 5 of the present disclosure;
FIG. 4 shows the results of TIGIT antibody H401LV2 (H401LV2-hIgG1, H401LV2-hIgG1LALA, H401LV2-hIgG4S228P) blocking the binding of CD155-mIgG2a antibody to 293T-TIGIT cells according to Example 6 of the present disclosure;
FIG. 5 shows the results of TIGIT antibody H401LV2-hIgG1LALA promoting IL-2 secretion by Jurkat T cells according to Example 7 of the present disclosure;
FIG. 6 shows the results of TIGIT antibody H401LV2-mIgG2a binding to CHO-K1-TIGIT cells according to Example 8 of the present disclosure;
FIG. 7 shows the results of TIGIT antibody H401LV2-mIgG2a blocking the binding of CD155 to CHO-K1-TIGIT cells according to Example 9 of the present disclosure;
FIG. 8 shows the results of the efficacy of TIGIT antibody H401LV2-mIgG2a in the colorectal cancer MC38 model according to Example 10 of the present disclosure; and
FIG. 9 shows the results of the efficacy of H401LV2-mIgG2aD265A in the lung cancer LLC model according to Example 10 of the present disclosure.

### DETAILED DESCRIPTION

The embodiments of the present disclosure are described in detail below, examples of which are illustrated in the accompanying drawings. The same or similar reference numerals represent the same or similar elements or elements having the same or similar functions throughout the accompanying drawings. The embodiments described below with reference to the accompanying drawings are exemplary and intended to explain the present disclosure, rather than to limit the present disclosure. The scope of protection is defined by the appended claims.

In describing the present disclosure, the terms related herein have been explained and illustrated. These explanations and illustrations are merely for the convenience of understanding the embodiments and are not to be construed as limiting the scope of the present disclosure.

As used herein, the terms "comprise" or "include" are open-ended expressions, meaning to include the content specified in the present disclosure, but not to exclude other aspects.

As used herein, the terms "optionally", "optional" or "option" generally mean that the subsequently described event or condition may or may not occur, and that the description includes both the case where the event or condition occurs and the case where it does not occur.

To facilitate a better understanding of the present disclosure, certain technical and scientific terms are specifically defined below. Unless otherwise clearly defined elsewhere in this document, all other technical and scientific terms used herein have the meanings commonly understood by those ordinarily skilled in the art to which this invention belongs. The abbreviations for amino acid residues are the standard 3-letter and/or 1-letter codes used in the art to denote one of the 20 common L-amino acids.

In the context of the present disclosure, unless stated to the contrary, the term "antigen-binding fragment" used, also referred to as "antibody fragment", generally refers to an antigen-binding antibody fragment, which may include a portion of an intact antibody, typically the antigen-binding region or variable region. Examples of antibody fragments include Fab, Fab', F(ab')2, Fv or scFv, diabodies, linear antibodies, single-chain antibody molecules, etc.

The terms "complementarity determining region" or "CDR" or "CDR sequence" refer to the amino acid sequences of an antibody that are responsible for antigen binding, which typically include, for example: amino acid residues near 23-34 (L1), 50-56 (L2) and 89-97 (L3) in the light chain variable region, and near 31-35B (H1), 50-65 (H2) and 95-102 (H3) in the heavy chain variable region (Kabat et al., Sequences of Proteins of Immunological Interest, 5th Ed. Public Health Service, National Institutes of Health, Bethesda, MD. (1991)); and/or amino acid residues from "hypervariable loops" (e.g., near 26-32 (L1), 50-52 (L2) and 91-96 (L3) in the light chain variable region, and near 26-32 (H1), 53-55 (H2) and 96-101 (H3) in the heavy chain variable region) (Chothia and Lesk, J. Mol. Biol. 196: 901-917 (1987)).

In the case of not substantially affecting the activity of the antibody (retaining at least 95% of the activity), those skilled in the art can substitute, add and/or delete one or more (e.g., 1, 2, 3, 4, 5, 6, 7, 8, 9 or 10 or more) amino acids to the sequences of the present disclosure to obtain variants of the sequences of the antibody or functional fragment thereof. They are all considered to be included within the scope of the present disclosure. For example, amino acids with similar properties are substituted in the variable region. The sequences of the variants of the present disclosure may have at least 80%, 85%, 90%, 95%, 96%, 97%, 98% or 99% identity (or homology) to a reference sequence. The sequence identity described in the present disclosure can be measured using sequence analysis software. For example, a computer program, BLAST with default parameters, particularly BLASTP or TBLASTN, is used. The amino acid sequences mentioned in the present disclosure are all presented from the N-terminus to the C-terminus.

As used herein, the terms "identity", "homology" or "similarity", when used to describe an amino acid sequence or nucleic acid sequence relative to a reference sequence, refer to the percentage of identical amino acids or nucleotides between two amino acid sequences or nucleic acid sequences as determined by conventional methods, see, e.g., Ausubel et al. (eds.)

(1995), Current Protocols in Molecular Biology, Chapter 19 (Greene Publishing and Wiley-Interscience, New York); and the ALIGN program (Dayhoff (1978), Atlas of Protein Sequence and Structure 5: Suppl.3 (National Biomedical Research Foundation, Washington, D.C.). There are many algorithms for aligning sequences and determining sequence identity, including the homology alignment algorithm of Needleman et al. (1970) J. Mol. Biol. 48: 443; the local homology algorithm of Smith et al. (1981) Adv. Appl. Math. 2: 482; the similarity search method of Pearson et al. (1988) Proc. Natl. Acad. Sci. 85: 2444; the Smith-Waterman algorithm (Meth. Mol. Biol. 70: 173-187 (1997)); and the BLASTP, BLASTN and BLASTX algorithms (see Altschul et al. (1990) J. Mol. Biol. 215: 403-410). Computer programs utilizing these algorithms are also available and include, but are not limited to: ALIGN or Megalign (DNASTAR) software, or WU-BLAST-2 (Altschul et al., Meth. Enzym., 266: 460-480 (1996)); or GAP, BESTFIT, BLAST (Altschul, et al., supra), FASTA and TFASTA, available in the Genetics Computing Group (GCG) package, Version 8, Madison, Wisconsin, USA; and CLUSTAL in the PC/Gene program provided by Intelligenetics, Mountain View, California.

As described above, the antibody of the present disclosure may be full-length (e.g., IgG1 or IgG4 antibodies) or may include only an antigen binding portion (e.g., Fab, F(ab')2 or scFv fragments), or may be modified to affect function. The present disclosure includes an anti-TIGIT antibody having a modified glycosylation pattern. In some applications, it may be useful to perform modifications in order to remove undesired glycosylation sites or to eliminate the presence of fucose moieties on the oligosaccharide chain in order to, for example, enhance antibody-dependent cellular cytotoxicity (ADCC) function. In other applications, galactosylation modifications can be performed to alter complement-dependent cytotoxicity (CDC).

The term "functional fragment" as used herein particularly refers to antibody fragments such as Fv, scFv (where "sc" means single chain), Fab, F(ab')2, Fab', scFv-Fc fragments or diabody, or any fragment that can increase the half-life through chemical modification (e.g., addition of poly(alkylene) glycol such as polyethylene glycol ("PEGylation") (PEGylated fragments referred to as Fv-PEG, scFv-PEG, Fab-PEG, F(ab')2-PEG or Fab'-PEG) ("PEG" refers to polyethylene glycol)) or incorporation into liposomes, provided that such fragments have TIGIT-binding activity. Preferably, the functional fragment will consist of or include partial sequences of the heavy chain variable region or the light chain variable region of the antibody from which it is derived, said partial sequences being sufficient to retain the same binding specificity and sufficient affinity as the antibody from which it is derived. For TIGIT, the affinity is preferably at least 1/100, and more preferably at least 1/10, of the antibody from which it is derived. Such a functional fragment will contain a minimum of 5 amino acids, preferably 10, 15, 25, 50 and 100 consecutive amino acids of the antibody sequence from which it is derived.

As described above, one aspect of the present disclosure provides an antibody or an antigen-binding fragment thereof. According to an embodiment of the present disclosure, the antibody or the antigen-binding fragment thereof includes:
a heavy chain variable region and a light chain variable region, wherein the amino acid sequence of the heavy chain variable region is as set forth in SEQ ID NO: 7, and the amino acid sequence of the light chain variable region is as set forth in SEQ ID NO: 8.

In a preferred embodiment of the present disclosure, to further improve the biological acceptability of the antibody, the antibody may further be humanized, i.e., the antibody is a chimeric antibody or a humanized antibody. The term "chimeric antibody" refers to a recombinant antibody obtained by replacing the constant region amino acid sequence of a monoclonal antibody from one species (e.g., mouse) with the constant region of an antibody from another species (e.g., human) using recombinant DNA technology. The term "humanized antibody" refers to a recombinant antibody obtained by replacing all of the non-CDR (Fv framework region (FR)) amino acid sequences of the constant and variable regions of a monoclonal antibody from one species (e.g., mouse) with the non-CDR amino acid sequences of the constant and variable regions of an antibody from another species (e.g., human) using recombinant DNA technology. That is to say, when the constant region of an antibody is humanized, it is referred to as a chimeric antibody, whereas when the non-CDR amino acid sequences of the constant and variable regions are all humanized, it is referred to as a humanized antibody. Humanization may be carried out by reference to conventional antibody engineering techniques and will not be described in detail herein.

In another aspect, the present disclosure provides a composition. According to an embodiment of the present disclosure, the composition includes: the above antibody or the antigen-binding fragment thereof, the nucleic acid molecule, the expression vector or the recombinant cell.

The composition provided by the present disclosure includes the antibody or the antigen-binding fragment thereof, the nucleic acid molecule, the expression vector, or the recombinant cell, as described above. In certain embodiments, the composition includes combinations that are separated in time and/or space, provided that they can act together to achieve the objectives of the present disclosure. For example, the components contained in the composition may be administered to a subject as a whole or separately. When the components contained in the composition are administered to a subject separately, each component may be administered to the subject simultaneously or sequentially.

The composition of the present disclosure may also be administered in combination with each other, or in combination with one or more other therapeutic compounds, e.g., in combination with chemotherapeutic agents. Therefore, the composition may further include a chemotherapeutic agent. The antibody or the antigen-binding fragment thereof, the nucleic acid molecule, the expression vector, or the recombinant cell according to the present disclosure may further be combined with a second therapeutic agent. Exemplary agents of the second therapeutic agent include, but are not limited to, other agents that inhibit TIGIT activity (including other antibodies or antigen-binding fragments thereof, peptide inhibitors, small-molecule antagonists, etc.) and/or agents that interfere with TIGIT upstream or downstream signal transduction.

Typically, the antibody or the antigen-binding fragment thereof is administered in an effective amount, i.e., an amount sufficient to achieve the desired therapeutic and/or prophylactic effect. For example, it refers to an amount that results in the prevention or amelioration of symptoms associated with the treated disease, such as a TIGIT-related disease. The effective amount of the composition administered to a subject will depend on the type and severity of the disease, as well as individual characteristics such as general health, age, gender, body weight and drug tolerance; it will also depend on the severity and type of the disease, and those skilled in the art can determine appropriate dosages based on these factors and others.

In yet another aspect, the present disclosure provides a medicament. According to an embodiment of the present disclosure, the medicament includes: the above antibody or the antigen-binding fragment thereof, the nucleic acid molecule, the expression vector, the recombinant cell, or the composition, and the medicament is for use in the treatment of a malignant tumor.

The medicament provided by the present disclosure includes a therapeutic agent and the antibody or the antigen-binding fragment thereof as described above, which is conjugated to the therapeutic agent. The antibody or the antigen-binding fragment thereof with the therapeutic agent can be conjugated using conventional methods.

In yet another aspect, the present disclosure provides a kit. According to an embodiment of the present disclosure, the kit includes the antibody or the antigen-binding fragment thereof according to the first aspect of the present disclosure.

In a further aspect, the present disclosure provides the use of the above antibody or the antigen-binding fragment thereof in the preparation of a kit. According to an embodiment of the present disclosure, the kit is for use in the detection of TIGIT.

The kit provided by the present disclosure for detecting TIGIT in a sample includes the antibody or the antigen-binding fragment thereof as described above. The sample may be tissue from patients with TIGIT-mediated diseases (particularly patients with transplant rejection, autoimmune diseases, infectious diseases or cancers, more preferably patients with at least one of lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, or head and neck cancer). The kit may further include reagents conventionally used for detecting TIGIT, such as a coating solution.

The present disclosure further provides use of the antibody or the antigen-binding fragment thereof as described above in the preparation of a reagent for detecting TIGIT in a sample. As described above, the sample may be tissue from patients with TIGIT-mediated diseases, which will not be described in detail herein. The antibody or the antigen-binding fragment thereof of the present disclosure has good affinity for TIGIT and can effectively detect TIGIT in the sample.

The present disclosure further provides the antibody or the antigen-binding fragment thereof for use in the prevention and/or treatment of a TIGIT-mediated disease. Preferably, the TIGIT-mediated disease includes transplant rejection, autoimmune diseases, infectious diseases or cancers. More preferably, the cancers are TIGIT-expressing cancers. Further preferably, the cancers are at least one of lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, or head and neck cancer. Further preferably, the infectious diseases include, but are not limited to, HIV infection and/or hepatitis B virus infection.

Those skilled in the art can clone a DNA molecule encoding the antibody or the antigen-binding fragment thereof of the present disclosure into a vector (particularly an expression vector), transform the vector into a host cell, and induce expression to obtain the antibody or the antigen-binding fragment thereof. Therefore, the present disclosure further provides a nucleic acid molecule encoding the above antibody or the antigen-binding fragment thereof, as well as a recombinant cell including the nucleic acid. The nucleic acid is preferably an expression cassette obtained by genetic engineering means.

The expression vector may refer to a cloning vector or a recombinant vector, and may be prepared by operably linking the nucleic acid with a commercially available vector (e.g., a plasmid or a viral vector). Common plasmids include pSeTag2, PEE14, pMH3, etc.

The expression vector of the present disclosure may contain DNA sequences encoding the heavy chain variable region, the light chain variable region and/or the constant region of the antibody. However, it is also possible to separately construct two expression vectors: one containing a heavy chain variable region and a heavy chain constant region, and the other containing a light chain variable region and a light chain constant region, for co-transfection into mammalian cells. In a preferred embodiment, the expression vector further contains a promoter, a DNA sequence encoding a secretion signal peptide, and at least one drug resistance gene for selection.

The recombinant cell described in the present disclosure may be a prokaryotic host cell, a eukaryotic host cell or a phage. The prokaryotic host cell may be *Escherichia coli, Bacillus subtilis, Streptomyces,* or *Proteus mirabilis,* etc. The eukaryotic host cell may be a fungus, such as *Pichia pastoris, Saccharomyces cerevisiae, Schizosaccharomyces, Trichoderma;* an insect cell, such as *Spodoptera frugiperda;* a plant cell, such as *Nicotiana tabacum;* or a mammalian cell, such as a BHK cell, CHO cell, COS cell, or myeloma cell. In some embodiments, the recombinant cell described in the present disclosure is preferably a mammalian cell, more preferably a BHK cell, CHO cell, NSO cell, or COS cell.

Nucleic acids encoding the heavy and/or light chains of the antibody of the present disclosure are within the scope of the present disclosure. The corresponding nucleotide sequences can be readily obtained by those skilled in the art based on the amino acid sequences of the heavy and/or light chains.

As used herein, "antibody affinity maturation" as described in the present disclosure refers to the normal state of immune function in the body. In humoral immunity, the phenomenon that the average affinity of antibodies produced in a secondary response is higher than in a primary immune response is referred to as antibody affinity maturation. This functional state of the body is the result of long-term evolution and continuous adaptation to the external environment, and is of great significance for the body's defense and maintenance of autoimmune surveillance. The present disclosure utilizes *in vitro* antibody affinity maturation technology. Specifically, it refers to a process of using molecular biology techniques to mutate the CDR amino acids of an antibody and screen from the mutated antibody library to obtain antibodies with significantly improved affinity.

As used herein, "H401LV2" refers to the TIGIT antibody prepared by the present disclosure.

As used herein, "Ka" has the same meaning as "kon" and refers to the association constant. "Kd" has the same meaning as "koff" and refers to the dissociation constant.

As used herein, "Jurkat cells" refer to a kind of suspension cells, an immortalized human T-lymphocyte cell line used for studying acute T-cell leukemia, T-cell signaling and the expression of various chemokine receptors susceptible to viral entry, particularly HIV.

The embodiments of the present disclosure will be described in more detail below, with examples of which are illustrated in the accompanying drawings. The embodiments described below with reference to the accompanying drawings are exemplary and intended to explain the present disclosure, rather than to limit the present disclosure.

It should be noted that "plasmid" and "vector" have the same meaning in the following examples and may be used interchangeably.

The parent antibody Hu4A501 used in the following examples refers to a humanized TIGIT antibody developed by the research group, and its sequence is specified in CN109384846B.

The Tiragolumab analog used refers to an analog of the anti-human TIGIT antibody Tiragolumab developed by Roche, which has the same amino acid sequence as Tiragolumab and was purchased from Biointron.

In the following examples, hIgG1 refers to the wild-type human IgG1 constant region, and antibodies of this type typically have ADCC (antibody-dependent cellular cytotoxicity) function; hIgG1LALA refers to the human IgG1 constant region with L234A and L235A mutations, which eliminate ADCC function; hIgG4S228P refers to the human IgG4 constant region with an S228P mutation, which is commonly performed in antibody engineering to eliminate the arm exchange phenomenon of wild-type IgG4.

### Example 1: Antibody Affinity Maturation

To improve the antibody drugability and obtain a high-affinity TIGIT antibody, the inventors performed affinity maturation on the developed humanized TIGIT antibody Hu4A501 (CN109384846B) (using the FASEBA technology, accomplished by GenScript Biotech). The antigen used for affinity maturation was a recombinant TIGIT extracellular domain-Fc fusion protein (TIGIT-Fc) (amino acid sequence as set forth in SEQ ID NO: 9). The heavy chain variable region and light chain variable region of the matured TIGIT antibody H401LV2 obtained are as set forth in SEQ ID NO: 7 and 8, respectively.

QVQLQESGPGLVKPSQTLSLTCTVTGSSITSDYAWNWIRQFPGKKLEWMG

YITYSGRTTYNPSLKSRITISRDTSKNQFSLKLSSVTAADTATYYCARWGLLRRYFDYW
GQGTLLTVSS (SEQ ID NO: 7),

DIVMTQSPSSMSTSVGDRVTITCRASQDVFNQVAWYQQKPGKSPKLLIYSA
SFRYTGVPDRFSGSGSGTDFTFTISSVQAEDFATYYCQQHYSTPLTFGAGTKLELK
(SEQ ID NO: 8),

MMTGTIETTGNISAEKGGSIILQCHLSSTTAQVTQVNWEQQDQLLAICNAD
LGWHISPSFKDRVAPGPGLGLTLQSLTVNDTGEYFCIYHTYPDGTYTGRIFLEVLELEP
KSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK
(SEQ ID NO: 9).

### Example 2: Antibody Affinity Assay

The Biacore method is a well-established objective assay for detecting the affinity and kinetics between proteins. The TIGIT antibody of the present disclosure was analyzed using Biacore T200 to characterize its affinity and binding kinetics.

The TIGIT extracellular domain-Fc fusion protein (TIGIT-Fc) was covalently attached to a CM5 (GE) chip using a standard amino coupling method. A series of TIGIT antibodies diluted in PBS buffer at a concentration gradient was then injected in cycles, followed by regeneration with 10 mM NaOH solution. The antigen-antibody binding kinetics were monitored for 3 minutes, followed by dissociation kinetics for 10 minutes. The resulting data were analyzed using GE's BIAevaluation software in a 1:1 (Langmuir) binding model. The Ka (kon), Kd (koff) and KD values determined by this method are shown in Table 1 below.

**Table 1: Affinity data of parental antibody (Hu4A501) and affinity-matured antibody H401LV2**

| | Ka (1/Ms) | Kd (1/s) | KD (M) |
|---|---|---|---|
| Hu4A501 | 9.89E+04 | 5.01E-04 | 5.06E-09 |
| h401LV2 | 1.97E+05 | 1.00E-06 | 5.08E-12 |

| | | | |
|---|---|---|---|
| Note: Ka represents the association constant (the larger the value, the stronger the affinity); Kd represents the dissociation constant (the smaller the value, the stronger the affinity), reflecting the affinity of a compound to a target; KD represents Kd/Ka and is the affinity constant. | | | |

### Example 3: ELISA Assay for TIGIT Antibody Binding

According to an embodiment of the present disclosure, the binding properties of the TIGIT antibodies were detected by the ELISA assay. The TIGIT extracellular domain-His tag fusion protein was coated onto a 96-well plate. After adding the antibodies, the binding properties between the antibodies and the TIGIT protein were determined based on the intensity of the signal. The experimental procedures were as follows:

### 1. Antibody Preparation

The pcDNA3.4 vectors containing the nucleotide sequences of the heavy and light chains of the above antibodies (synthesized by Nanjing GenScript, Hu4A501 light chain or Hu4A501-hK (amino acid sequence as set forth in SEQ ID NO: 17), Hu4A501 heavy chain or H401LV2-hIgG1 (amino acid sequence as set forth in SEQ ID NO: 18), H401LV2 light chain or H401LV2-hK (amino acid sequence as set forth in SEQ ID NO: 19), H401LV2 heavy chain or H401LV2-hIgG1 (amino acid sequence as set forth in SEQ ID NO: 20), H401LV2-hIgG1LALA (amino acid sequence as set forth in SEQ ID NO: 21), H401LV2-hIgG4S228P (amino acid sequence as set forth in SEQ ID NO: 22)) were transiently transfected into ExpiCHO-S cells (Gibco, Cat. No. A29127) to prepare the antibodies.

DIVMTQSPSSMSTSVGDRVTITCRASQDVFTAVAWYQQKPGKSPKLLIYSA
SFRYTGVPDRFSGSGSGTDFTFTISSVQAEDFATYYCQQHYSTPLTFGAGTKLELKRTV
AAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC (SEQ ID NO: 17)

QVQLQESGPGLVKPSQTLSLTCTVTGSSITSDYAWNWIRQFPGKKLEWMG
YITYSGRTTYNPSLKSRITISRDTSKNQFSLKLSSVTAADTATYYCARWGLLRRYFDYW
GQGTLLTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISK
AKGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK (SEQ ID
NO: 18)

DIVMTQSPSSMSTSVGDRVTITCRASQDVFNQVAWYQQKPGKSPKLLIYSA
SFRYTGVPDRFSGSGSGTDFTFTISSVQAEDFATYYCQQHYSTPLTFGAGTKLELKRTV
AAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC (SEQ ID NO: 19)

QVQLQESGPGLVKPSQTLSLTCTVTGSSITSDYAWNWIRQFPGKKLEWMG
YITYSGRTTYNPSLKSRITISRDTSKNQFSLKLSSVTAADTATYYCARWGLLRRYFDYW
GQGTLLTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISK
AKGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK (SEQ ID
NO: 20)

QVQLQESGPGLVKPSQTLSLTCTVTGSSITSDYAWNWIRQFPGKKLEWMG
YITYSGRTTYNPSLKSRITISRDTSKNQFSLKLSSVTAADTATYYCARWGLLRRYFDYW
GQGTLLTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVEPKSCDKT
HTCPPCPAPEAAGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISK
AKGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK (SEQ ID
NO: 21)

QVQLQESGPGLVKPSQTLSLTCTVTGSSITSDYAWNWIRQFPGKKLEWMG
YITYSGRTTYNPSLKSRITISRDTSKNQFSLKLSSVTAADTATYYCARWGLLRRYFDYW
GQGTLLTVSSASTKGPSVFPLAPCSRSTSESTAALGCLVKDYFPEPVTVSWNSGALTSG
VHTFPAVLQSSGLYSLSSVVTVPSSSLGTKTYTCNVDHKPSNTKVDKRVESKYGPPCP
PCPAPEFEGGPSVFLFPPKPKDTLMISRTPEVTCVVVDVSQEDPEVQFNWYVDGVEVH
NAKTKPREEQFNSTYRVVSVLTVLHQDWLNGKEYKCKVSNKGLPSSIEKTISKAKGQ
PREPQVYTLPPSQEEMTKNQVSLTCLVKGFYPSDIAVEWESNGQPENNYKTTPPVLDS
DGSFFLYSRLTVDKSRWQEGNVFSCSVMHEALHNHYTQKSLSLSLGK (SEQ ID NO: 22)

One day before transfection, ExpiCHO-S cells were adjusted to a density of (3 to 4) × 10⁶ cells/mL and cultured overnight with shaking at 37°C, 8% CO₂ and 95 rpm. On the day of transfection, when the cells had grown to 7 × 10⁶ to 1 × 10⁷ cells/mL with a viability of greater than 95%, it was ready for transfection. The cells were diluted to 6 × 10⁶ cells/mL using freshly pre-warmed ExpiCHO medium (Gibco, Cat. No. A2910002). The above pcDNA 3.4 plasmids carrying the heavy and light chains (at a 1:1 ratio of light to heavy chain plasmids) and ExpiFectamine CHO transfection reagent (Gibco, Cat. No. A29129) were transfected into ExpiCHO-S cells and cultured with shaking at 37°C, 8% CO₂ and 95 rpm. 18-22 h after transfection, ExpiFectamine CHO Enhancer and ExpiCHO Feed were mixed well and then immediately added to the transfected cells. After mixing well, the cells were cultured with shaking at 32°C, 5% CO₂ and 95 rpm. On day 5 after transfection, the cells were supplemented with an additional 8 mL of ExpiCHO Feed, mixed well, and then cultured continuously. The changes in cell number and cell viability were observed daily. The cells were harvested by centrifugation when the cell viability dropped below 80% or after 10 to 14 days of culture. The expressed supernatant was filtered through a 0.45 µm filter membrane. Antibodies with Fc domains were captured from the expressed supernatant using a Mabselect prism A protein A affinity chromatography column (purchased from Suzhou Nanomicro Technology Co., Ltd.). After equilibrating the affinity chromatography column with phosphate buffer (pH 7.2), the supernatant was loaded onto the column. The antibodies were eluted with elution buffer (100 mM citric acid, pH 2.7) and finally concentrated and exchanged into PBS buffer. The purity of the purified antibodies was confirmed to be above 95% by SDS-PAGE. The results showed that the above antibodies were finally obtained.

### 2. ELISA Assay for Binding Properties of TIGIT Antibody

1. Human and cynomolgus monkey TIGIT-His tag fusion proteins (purchased from Acro) were diluted to 2 µg/ml with PBS buffer, added to a 96-well plate at 100 µl/well, and placed at 4°C overnight.
2. The PBS buffer in the 96-well plate from step 1 was removed, and the plate was washed 6 times with PBST buffer (pH 7.2; containing 0.1% of the total buffer volume of Tween 20). Then, PBS buffer (containing 10% by volume of BSA (bovine serum albumin)) was added at 200 µl/well, and the plate was incubated at 37°C for 2 h for blocking.
3. The PBS buffer from step 2 was removed, and the plate was washed 6 times with PBST buffer. Then, the test TIGIT antibodies diluted to 10,000, 2,000, 400, 80, 16, 3.2, 0.64 and 0.128 ng/ml with PBST buffer (containing 0.05% by volume of BSA) was added at 100 µl/well, and the plate was incubated at 37°C for 1 h.
4. The buffer from step 3 was removed, and the plate was washed 6 times with PBST. Then, HRP (horseradish peroxidase)-labeled anti-human IgG antibody (secondary antibody, purchased from Jacksonlab) diluted with PBST buffer (containing 0.05% by volume of BSA) was added at 100 µl/well, and the plate was incubated at 37°C for 1 h.
5. The 96-well plate from step 4 was washed 6 times with PBST buffer. Then, TMB (tetramethylbenzidine) was added at 80 µl/well, and the plate was incubated at room temperature for 3 min. Then, 4 M sulfuric acid was added at 80 µl/well to terminate the reaction.
6. The absorbance of the 96-well plate from step 5 was read at 450 mm using a microplate reader.

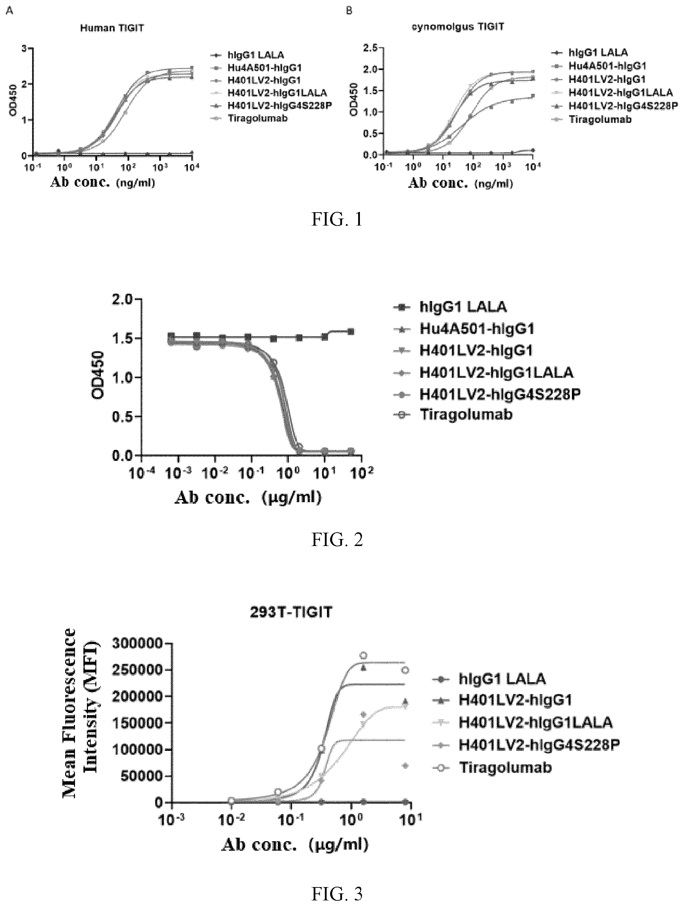

As shown in FIG. 1, the results demonstrate that the TIGIT antibody H401LV2 (H401LV2-hIgG1, H401LV2-hIgG1LALA, H401LV2-hIgG4S228P) prepared by the present disclosure can bind to TIGIT protein with stronger binding than the parental antibody Hu4A501 and the Tiragolumab analog (purchased from Biointron).

### Example 4: ELISA Assay for TIGIT Antibody Blocking

According to an embodiment of the present disclosure, the binding properties of the TIGIT antibodies were detected by the ELISA assay. The TIGIT extracellular domain-His tag fusion protein was coated onto a 96-well plate. After adding the antibodies and CD155-Biotin, the ability of the antibodies to block the binding of TIGIT/CD155 was determined based on the intensity of the signal. The specific procedures were as follows:
1. Human and cynomolgus monkey TIGIT-His tag fusion proteins (purchased from Acro) were diluted to 2 µg/ml with PBS buffer, added to a 96-well plate at 100 µl/well, and placed at 4°C overnight.
2. The PBS buffer in the 96-well plate from step 1 was removed, and the plate was washed 6 times with PBST buffer (pH 7.2; containing 0.1% by volume of Tween 20). Then, PBS buffer/(containing 10% by volume of BSA (bovine serum albumin)) 10% BSA was added at 200 µl/well, and the plate was incubated at 37°C for 2 h for blocking.
3. The PBS blocking buffer from step 2 was removed, and the plate was washed 6 times with PBST buffer. Then, the test TIGIT antibodies diluted to an appropriate concentration with PBST buffer (containing 0.05% by volume of BSA)/0.05% BSA was added at 100 µl/well, and the plate was incubated at 37°C for 1 h.
4. The buffer in the reaction system from step 3 was removed, and the plate was washed 6 times with PBST. Then, HRP (horseradish peroxidase)-labeled anti-human IgG antibody (secondary antibody, purchased from Jacksonlab) diluted with PBST buffer (containing 0.05% by volume of BSA) PBST/0.05% BSA was added at 100 µl/well, and the plate was incubated at 37°C for 1 h.
5. The 96-well plate from step 4 was washed 6 times with PBST buffer. Then, TMB (tetramethylbenzidine) was added at 80 µl/well, and the plate was incubated at room temperature for 3 min. Then, 4 M sulfuric acid was added at 80 µl/well to terminate the reaction.
6. The absorbance of the 96-well plate from step 5 was read at 450 mm using a microplate reader.

As shown in FIG. 2, the results demonstrate that the TIGIT antibody H401LV2 (H401LV2-hIgG1, H401LV2-hIgG1LALA, H401LV2-hIgG4S228P) prepared by the present disclosure can block the binding of TIGIT/CD155, with blocking capacity no weaker than that of the Tiragolumab analog (purchased from Biointron).

### Example 5: Binding Assay of TIGIT Antibody to 293T-TIGIT

According to an embodiment of the present disclosure, the binding properties of the TIGIT antibodies were detected by flow cytometry. The TIGIT protein was overexpressed in HEK293T cells (ATCC No. CRL-3216) and was designated as 293T-TIGIT. After adding the antibodies, the binding properties of the chimeric antibodies to TIGIT were determined based on the intensity of the signal. The specific procedures were as follows:
1. HEK293T cells were seeded into a 6-well plate at 5 × 10⁵ cells/well and cultured overnight in DMEM medium without antibiotics.
2. The medium was discarded before transfection, and 1 ml of fresh DMEM medium without antibiotics was added. The pLVX-EF1a-TIGIT-IRES-puro vector (pLVX-EFla-IRES-puro vector with TIGIT protein sequence (SEQ ID NO: 11) inserted between the EcoRI and BamHI restriction sites), pMD2G vector, and psPAX2 vector (3 µg in total for all three vectors) were added at a ratio of 2:1:1 to 200 µl of serum-free DMEM medium. 
MRWCLLLIWAQGLRQAPLASGMMTGTIETTGNISAEKGGSIILQCHLSSTT
AQVTQVNWEQQDQLLAICNADLGWHISPSFKDRVAPGPGLGLTLQSLTVNDTGEYFC
IYHTYPDGTYTGRIFLEVLESSVAEHGARFQIPLLGAMAATLVVICTAVIVVVALTRKK
KALRIHSVEGDLRRKSAGQEEWSPSAPSPPGSCVQAEAAPAGLCGEQRGEDCAELHD
YFNVLSYRSLGNCSFFTETG (SEQ ID NO: 11).
3. To the medium from step 2, 12 µg of polyetherimide (PEI, Polysciences, Inc.) was added. After mixing well, the mixture was allowed to stand for 16 min, and then the entire liquid was added to a 6-well plate containing HEK293T cells. The plate was incubated for 6 h.
4. The medium in the 6-well plate was discarded, and fresh complete DMEM medium was added for culture. After 48 h of transfection, the cell culture supernatant was harvested and filtered through a 0.45 µm filter (purchased from Millipore) to obtain the viral supernatant.
5. The entire virus supernatant from step 4 was added to a 6-well plate containing 1 × 10⁴ HEK293T cells, and polybrene (purchased from Sigma) was added at a final concentration of 4 µg/ml. The plate was incubated for 12 h.
6. The supernatant from step 5 was completely discarded, and fresh complete DMEM medium was added. The cultured cells were 293T-TIGIT cells.
7. The 293T-TIGIT cells were diluted to 2 × 10⁶ cells/ml with PBS and added to 1.5-ml EP tubes at 100 µl/tube. Then, mouse serum was added at 10 µl/tube, and the cells were blocked at 4°C for 30 min.
8. To the EP tubes from step 7, TIGIT antibodies at different concentration gradients (starting from 8 µg/ml with 5-fold serial dilutions, a total of 8 gradients) were added, followed by incubation at 4°C for 30 min.
9. To the EP tubes from step 8, 1 ml of PBS was added. The tubes were centrifuged at 3500 rpm for 5 min at 4°C, and the supernatant was completely discarded. The pellets were washed once more with PBS. After centrifugation, the supernatant was completely discarded. The cells were resuspended in 100 µl/tube of PBS, and Alexa-647-labeled mouse anti-human Fc antibody (secondary antibody, purchased from Jacksonlab) was added at 1 µl/tube, followed by incubation at 4°C in the dark for 30 min.
10. The cells from step 9 were washed twice with PBS and centrifuged. The supernatant was then completely discarded. The cells were resuspended in 200 µl/tube of PBS buffer and detected by flow cytometry.

As shown in FIG. 3, the results demonstrate that the TIGIT antibody H401LV2 (H401LV2-hIgG1, H401LV2-hIgG1LALA, H401LV2-hIgG4S228P) of the present disclosure can bind to 293T-TIGIT.

### Example 6: Blocking Assay of TIGIT Antibody

The TIGIT antibody blocks the binding of TIGIT to its ligand CD155 by binding to the extracellular domain of TIGIT. In this example of the present disclosure, the blocking effect of the TIGIT antibodies on CD155 binding to 293T-TIGIT was detected by flow cytometry. The specific procedures were as follows:
1. The 293T-TIGIT cells (as in Example 5) were diluted to 2 × 10⁶ cells/ml with PBS and added to 1.5-ml EP tubes at 100 µl/tube. Then, mouse serum was added at 10 µl/tube, and the cells were blocked at 4°C for 30 min.
2. To the EP tubes from step 1, TIGIT antibodies with 5-fold serial dilutions starting from 200 µg/ml (10 dilution gradients in total) and CD155-mIgG2a-Fc (purchased from Acro) were added, followed by incubation at 4°C for 30 min.
3. To EP tubes from step 2, APC-labeled goat anti-mouse IgG2a secondary antibody (purchased from Biolegend) was added at 0.1 µg/tube, followed by incubation at 4°C for 30 min. The cells were washed twice with PBS and centrifuged. The supernatant was then completely discarded. The cells were resuspended in 200 µl/tube of PBS and detected by flow cytometry.

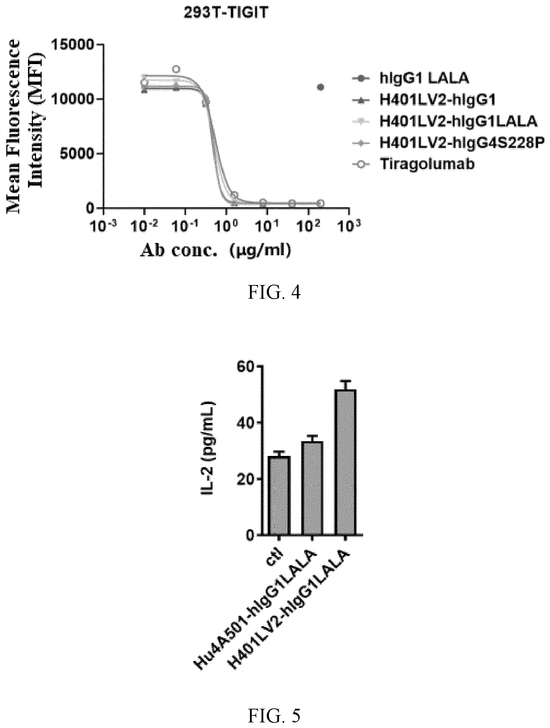

As shown in FIG. 4, the results demonstrate that the TIGIT antibody H401LV2 (H401LV2-hIgG1, H401LV2-hIgG1LALA, H401LV2-hIgG4S228P) containing different constant regions of the present disclosure can block the binding of CD155-mIgG2a to 293T-TIGIT, with blocking capacity no weaker than that of the Tiragolumab analog (purchased from Biointron).

### Example 7: Assay for TIGIT Antibody Promoting Jurkat T Cell Activation

Jurkat cells were co-incubated with U-937 tumor cells, and CD3 antibody and TIGIT antibodies were added to detect the content of IL-2 in the supernatant. The activation effect of Jurkat cells was determined based on the intensity of IL-2 signaling after antibody addition. The specific procedures were as follows:
1. The OKT3 antibody (purchased from Biolegend) was diluted to 0.2 µg/ml with PBS and added to a 96-well plate for overnight reaction. The supernatant was then discarded.
2. Jurkat cells were diluted to 1 × 10⁶ cells/ml with complete 1640 medium and added to the 96-well plate at 100 µl/tube; U-937 cells were diluted to 1.25 × 10⁶ cells/ml with complete 1640 medium and added to the 96-well plate at 80 µl/tube; the TIGIT antibodies were diluted to 100 µg/ml with complete 1640 medium and added to the 96-well plate at 20 µl/well. The plate was incubated at 37°C in a 5% CO₂ incubator for 24 h. The IL-2 content in the supernatant was detected using the CBA kit (purchased from BD).

As shown in FIG. 5, the results demonstrate that the affinity-matured TIGIT antibody H401LV2-hIgG1LALA of the present disclosure exhibits superior ability to promote Jurkat T cell activation compared to the parental antibody Hu4A501-hIgG1LALA.

### Example 8: Binding Assay of TIGIT Antibody to CHO-K1-TIGIT

In this example of the present disclosure, the binding properties of TIGIT antibodies were detected by flow cytometry. The TIGIT protein was overexpressed in CHO-K1 cells (ATCC No. CCL-61) and designated as CHO-K 1-TIGIT. The binding properties of the chimeric antibodies to TIGIT were determined based on the intensity of the signal after antibody addition.
1. Antibody Preparation: The pcDNA3.4 vectors containing the nucleotide sequences of the heavy and light chains of the above antibodies (synthesized by Nanjing GenScript, 4.1D3 light chain or 4.1D3-mK (amino acid sequence as set forth in SEQ ID NO: 10), 4.1D3 heavy chain or 4.1D3-mIgG2a (amino acid sequence as set forth in SEQ ID NO: 11), 22G2 light chain or 22G2-mK (amino acid sequence as set forth in SEQ ID NO: 12), 22G2 heavy chain or 22G2-mIgG2a (amino acid sequence as set forth in SEQ ID NO: 13), H401LV2 light chain or H401LV2-mK (amino acid sequence as set forth in SEQ ID NO: 14), H401LV2 heavy chain or H401LV2-mIgG2a (amino acid sequence as set forth in SEQ ID NO: 15)) were transiently transfected into ExpiCHO-S cells (Gibco, Cat. No. A29127) to prepare the antibodies. 
DIVMTQSPDSLAVSLGERATINCKSSQTVLYSSNNKKYLAWYQQKPGQPPN
LLIYWASTRESGVPDRFSGSGSGTDFTLTISSLQAEDVAVYYCQQYYSTPFTFGPGTKV
 
EIKRADAAPTVSIFPPSSEQLTSGGASVVCFLNNFYPKDINVKWKIDGSERQNGVLNS
WTDQDSKDSTYSMSSTLTLTKDEYERHNSYTCEATHKTSTSPIVKSFNRNEC (SEQ ID
NO: 10),
 
EVQLQQSGPGLVKPSQTLSLTCAISGDSVSSNSAAWNWIRQSPSRGLEWLG
KTYYRFKWYSDYAVSVKGRITINPDTSKNQFSLQLNSVTPEDTAVFYCTRESTTYDLL
AGPFDYWGQGTLVTVSSAKTTAPSVYPLAPVCGDTTGSSVTLGCLVKGYFPEPVTLT
WNSGSLSSGVHTFPAVLQSDLYTLSSSVTVTSSTWPSQSITCNVAHPASSTKVDKKIEP
RGPTIKPCPPCKCPAPNLLGGPSVFIFPPKIKDVLMISLSPIVTCVVVAVSEDDPDVQISW
FVNNVEVHTAQTQTHREDYNSTLRVVSALPIQHQDWMSGKEFKCKVNNKDLPAPIER
TISKPKGSVRAPQVYVLPPPEEEMTKKQVTLTCMVTDFMPEDIYVEWTNNGKTELNY
KNTEPVLDSDGSYFMYSKLRVEKKNWVERNSYSCSVVHEGLHNHHTTKSFSRTPGK
(SEQ ID NO: 11),
 
EIVLTQSPATLSLSPGERATLSCRASQSVSSYLAWYQQKPGQAPRLLIYDAS
NRATGIPARFSGSGSGTDFTLTISSLEPEDFAVYYCQQRSNWPPLFTFGPGTKVDIKRAD
AAPTVSIFPPSSEQLTSGGASVVCFLNNFYPKDINVKWKIDGSERQNGVLNSWTDQDS
KDSTYSMSSTLTLTKDEYERHNSYTCEATHKTSTSPIVKSFNRNEC (SEQ ID NO: 12),
 
QVQLQESGPGLVKPSETLSLTCTVSGGSVSSGIYYWSWIRQPPGKGLEWIG
YIYYSGSTNYNPSLKSRVTISVDTSKNQFSLKLSSVTAADTAVYYCARDYYVSGNYYN
VDYYFFGVDVWGQGTTVTVSSAKTTAPSVYPLAPVCGDTTGSSVTLGCLVKGYFPEP
VTLTWNSGSLSSGVHTFPAVLQSDLYTLSSSVTVTSSTWPSQSITCNVAHPASSTKVDK
KIEPRGPTIKPCPPCKCPAPNLLGGPSVFIFPPKIKDVLMISLSPIVTCVVVAVSEDDPDV
QISWFVNNVEVHTAQTQTHREDYNSTLRVVSALPIQHQDWMSGKEFKCKVNNKDLP
APIERTISKPKGSVRAPQVYVLPPPEEEMTKKQVTLTCMVTDFMPEDIYVEWTNNGK
TELNYKNTEPVLDSDGSYFMYSKLRVEKKNWVERNSYSCSVVHEGLHNHHTTKSFS
RTPGK (SEQ ID NO: 13).
 
DIVMTQSPSSMSTSVGDRVTITCRASQDVFNQVAWYQQKPGKSPKLLIYSA
SFRYTGVPDRFSGSGSGTDFTFTISSVQAEDFATYYCQQHYSTPLTFGAGTKLELKRAD
AAPTVSIFPPSSEQLTSGGASVVCFLNNFYPKDINVKWKIDGSERQNGVLNSWTDQDS
KDSTYSMSSTLTLTKDEYERHNSYTCEATHKTSTSPIVKSFNRNEC (SEQ ID NO: 14)
 QVQLQESGPGLVKPSQTLSLTCTVTGSSITSDYAWNWIRQFPGKKLEWMG 
YITYSGRTTYNPSLKSRITISRDTSKNQFSLKLSSVTAADTATYYCARWGLLRRYFDYW
GQGTLLTVSSAKTTAPSVYPLAPVCGDTTGSSVTLGCLVKGYFPEPVTLTWNSGSLSS
GVHTFPAVLQSDLYTLSSSVTVTSSTWPSQSITCNVAHPASSTKVDKKIEPRGPTIKPCP
PCKCPAPNLLGGPSVFIFPPKIKDVLMISLSPIVTCVVVDVSEDDPDVQISWFVNNVEV
HTAQTQTHREDYNSTLRVVSALPIQHQDWMSGKEFKCKVNNKDLPAPIERTISKPKGS
VRAPQVYVLPPPEEEMTKKQVTLTCMVTDFMPEDIYVEWTNNGKTELNYKNTEPVL
DSDGSYFMYSKLRVEKKNWVERNSYSCSVVHEGLHNHHTTKSFSRTPGK (SEQ ID
NO: 15)
 One day before transfection, ExpiCHO-S cells were adjusted to a density of (3 to 4) × 10⁶ cells/mL and cultured overnight with shaking at 37°C, 8% CO₂ and 95 rpm. On the day of transfection, when the cells had grown to 7 × 10⁶ to 1 × 10⁷ cells/mL with a viability of greater than 95%, it was ready for transfection. The cells were diluted to 6 × 10⁶ cells/mL using freshly pre-warmed ExpiCHO medium (Gibco, Cat. No. A2910002). The above pcDNA 3.4 plasmids carrying the heavy and light chains (at a 1:1 ratio of light to heavy chain plasmids) and ExpiFectamine CHO transfection reagent (Gibco, Cat. No. A29129) were transfected into ExpiCHO-S cells and cultured with shaking at 37°C, 8% CO₂ and 95 rpm. 18-22 h after transfection, ExpiFectamine CHO Enhancer and ExpiCHO Feed were mixed well and then immediately added to the transfected cells. After mixing well, the cells were cultured overnight with shaking at 32°C, 5% CO₂ and 95 rpm. On day 5 after transfection, the cells were supplemented with an additional 8 mL of ExpiCHO Feed, mixed well, and then cultured continuously. The changes in cell number and cell viability were observed daily. The cells were harvested by centrifugation when the cell viability dropped below 80% or after 10 to 14 days of culture. The expressed supernatant was filtered through a 0.45 µm filter membrane. Antibodies with Fc domains were captured from the expressed supernatant using a Mabselect prism A protein A affinity chromatography column (purchased from Suzhou Nanomicro Technology Co., Ltd.). After equilibrating the affinity chromatography column with phosphate buffer (pH 7.2), the supernatant was loaded onto the column. The antibodies were eluted with elution buffer (100 mM citric acid, pH 2.7) and finally concentrated and exchanged into PBS buffer. The purity of the purified antibodies was confirmed to be above 95% by SDS-PAGE. The results showed that the above antibodies were finally obtained.
2. Flow Cytometry Assay for Binding Properties of Chimeric Antibodies To TIGIT

1. HEK293T cells were seeded into a 6-well plate at 5 × 10⁵ cells/well and cultured overnight in DMEM medium without antibiotics.
2. The medium was discarded before transfection, and 1 ml of fresh DMEM medium without antibiotics was added. The pLVX-EF1a-TIGIT-IRES-puro vector (pLVX-EFla-IRES-puro vector with TIGIT protein inserted between the EcoRI and BamHI restriction sites), pMD2G vector, and psPAX2 vector (3 µg in total for all three vectors) were added at a ratio of 2:1:1 to 200 µl of serum-free DMEM medium.
3. To the medium from step 2, 12 µg of polyetherimide (PEI, Polysciences, Inc.) was added. After mixing well, the mixture was allowed to stand for 16 min, and then the entire liquid was added to a 6-well plate containing CHO-K1 cells.
4. The 6-well plate from step 3 was incubated for 6 h. The medium was discarded, and fresh complete DMEM medium was added for culture.
5. After 48 h of transfection, the cell culture supernatant was harvested and filtered through a 0.45 µm filter (purchased from Millipore) to obtain a filtrate as the viral supernatant.
6. The entire virus supernatant was added to a 6-well plate containing 1 × 10⁴ 293 T cells, and polybrene (purchased from Sigma) was added at a final concentration of 4 µg/ml. The plate was incubated for 12 h.
7. The supernatant was completely discarded, and fresh complete DMEM medium was added for culture. The cultured cells were CHO-K1-TIGIT cells.
8. The CHO-K1-TIGIT cells were diluted to 2 × 10⁶ cells/ml with PBS and added to 1.5-ml EP tubes at 100 µl/tube. Then, mouse serum was added at 10 µl/tube, and the cells were blocked in a 4°C refrigerator for 30 min.
9. To the EP tubes from step 8, TIGIT antibodies at different concentration gradients were added, followed by incubation in a 4°C refrigerator for 30 min. To the EP tubes, 1 ml of PBS was added. The tubes were centrifuged at 3500 rpm for 5 min at 4°C, and the supernatant was completely discarded.
10. The cells were resuspended in PBS again. After centrifugation, the supernatant was completely discarded. The cells were resuspended in 100 µl/tube of PBS, and Alexa-647-labeled mouse anti-human Fc antibody (secondary antibody, purchased from Jacksonlab) was added at 1 µl/tube, followed by incubation at 4°C in the dark for 30 min. The cells were washed twice with PBS and centrifuged. The supernatant was then completely discarded.
11. The cells were resuspended in 200 µl/tube of PBS and then detected by flow cytometry.

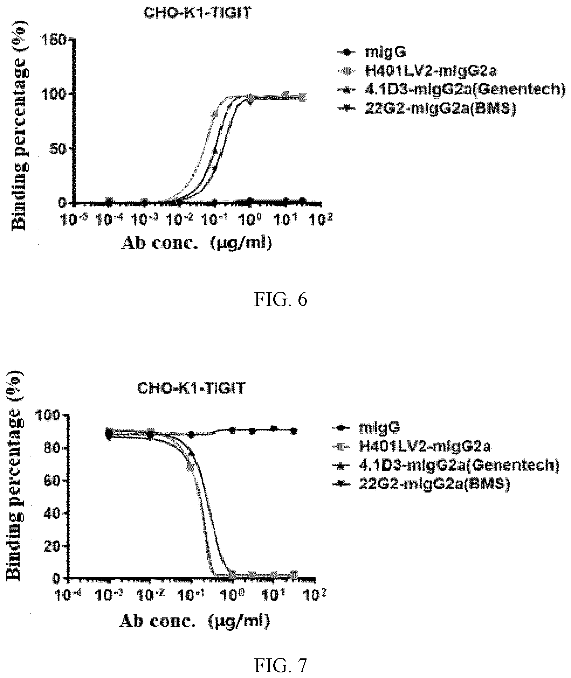

As shown in FIG. 6, the results demonstrate that the TIGIT antibody H401LV2-mIgG2a of the present disclosure can bind to CHO-K1-TIGIT with a binding capacity superior to that of the 4.1D3 and 22G2 antibodies.

### Example 9: Blocking Assay of TIGIT Antibody

TIGIT antibodies block the binding of TIGIT to its ligand CD155 by binding to the extracellular domain of TIGIT. In the present disclosure, the blocking effect of the TIGIT antibodies on CD155 binding to CHO-K1-TIGIT was detected by flow cytometry. The specific procedures were as follows:
1. The CHO-K1-TIGIT cells (as in Example 8) were diluted to 2 × 10⁶ cells/ml with PBS and added to 1.5-ml EP tubes at 100 µl/tube. Then, mouse serum was added at 10 µl/tube, and the cells were blocked at 4°C for 30 min.
2. TIGIT antibodies (30, 10, 3, 1, 0.1, 0.01, 0.001 µg/ml) and CD155-mIgG2a-Fc (purchased from Acro) were added, followed by incubation at 4°C for 30 min. APC-labeled goat anti-mouse IgG2a (secondary antibody, purchased from Biolegend) was added at 0.1 µg/tube, followed by incubation at 4°C for 30 min.
3. The cells were washed twice with PBS and centrifuged. The supernatant was then completely discarded. The cells were resuspended in 200 µl/tube of PBS and finally detected by flow cytometry.

As shown in FIG. 7, the results demonstrate that the antibody H401LV2-mIgG2a of the present disclosure can block the binding of CD155 to CHO-K1-TIGIT with a stronger blocking capacity than the 4.1D3 antibody.

### Example 10: Anti-Tumor Effect of H401LV2 Antibody in Mice

The effect of the affinity-matured TIGIT antibody H401LV2 on anti-tumor function in hTIGIT transgenic mice was detected using an *in vivo* efficacy assay. The specific procedures were as follows:

### 1. Antibody Preparation

The pcDNA3.4 vectors containing the nucleotide sequences of the heavy and light chains of the above antibodies (synthesized by Nanjing GenScript, H401LV2 light chain or H401LV2-mK (amino acid sequence as set forth in SEQ ID NO: 14), H401LV2 heavy chain or H401LV2-mIgG2a (amino acid sequence as set forth in SEQ ID NO: 15), H401LV2-mIgG2aD265A (amino acid sequence as set forth in SEQ ID NO: 16)) were transiently transfected into ExpiCHO-S cells (Gibco, Cat. No. A29127) to prepare the antibodies.

QVQLQESGPGLVKPSQTLSLTCTVTGSSITSDYAWNWIRQFPGKKLEWMG
YITYSGRTTYNPSLKSRITISRDTSKNQFSLKLSSVTAADTATYYCARWGLLRRYFDYW
GQGTLLTVSSAKTTAPSVYPLAPVCGDTTGSSVTLGCLVKGYFPEPVTLTWNSGSLSS
GVHTFPAVLQSDLYTLSSSVTVTSSTWPSQSITCNVAHPASSTKVDKKIEPRGPTIKPCP
PCKCPAPNLLGGPSVFIFPPKIKDVLMISLSPIVTCVVVAVSEDDPDVQISWFVNNVEV
HTAQTQTHREDYNSTLRVVSALPIQHQDWMSGKEFKCKVNNKDLPAPIERTISKPKGS
VRAPQVYVLPPPEEEMTKKQVTLTCMVTDFMPEDIYVEWTNNGKTELNYKNTEPVL
DSDGSYFMYSKLRVEKKNWVERNSYSCSVVHEGLHNHHTTKSFSRTPGK (SEQ ID
NO: 16)

One day before transfection, ExpiCHO-S cells were adjusted to a density of (3 to 4) × 10⁶ cells/mL and cultured overnight with shaking at 37°C, 8% CO₂ and 95 rpm. On the day of transfection, when the cells had grown to 7 × 10⁶ to 1 × 10⁷ cells/mL with a viability of greater than 95%, it was ready for transfection. The cells were diluted to 6 × 10⁶ cells/mL using freshly pre-warmed ExpiCHO medium (Gibco, Cat. No. A2910002). The above pcDNA 3.4 plasmids carrying the heavy and light chains (at a 1:1 ratio of light to heavy chain plasmids) and ExpiFectamine CHO transfection reagent (Gibco, Cat. No. A29129) were transfected into ExpiCHO-S cells and cultured with shaking at 37°C, 8% CO₂ and 95 rpm. 18-22 h after transfection, ExpiFectamine CHO Enhancer and ExpiCHO Feed were mixed well and then immediately added to the transfected cells. After mixing well, the cells were cultured with shaking at 32°C, 5% CO₂ and 95 rpm. On day 5 after transfection, the cells were supplemented with an additional 8 mL of ExpiCHO Feed, mixed well, and then cultured continuously. The changes in cell number and cell viability were observed daily. The cells were harvested by centrifugation when the cell viability dropped below 80% or after 10 to 14 days of culture. The expressed supernatant was filtered through a 0.45 µm filter membrane. Antibodies with Fc domains were captured from the expressed supernatant using a Mabselect prism A protein A affinity chromatography column (purchased from Suzhou Nanomicro Technology Co., Ltd.). After equilibrating the affinity chromatography column with phosphate buffer (pH 7.2), the supernatant was loaded onto the column. The antibodies were eluted with elution buffer (100 mM citric acid, pH 2.7) and finally concentrated and exchanged into PBS buffer. The purity of the purified antibodies was confirmed to be above 95% by SDS-PAGE. The results showed that the above antibodies were finally obtained.

### 2. Detection of the effect of H401LV2 on anti-tumor function in hTIGIT transgenic mice

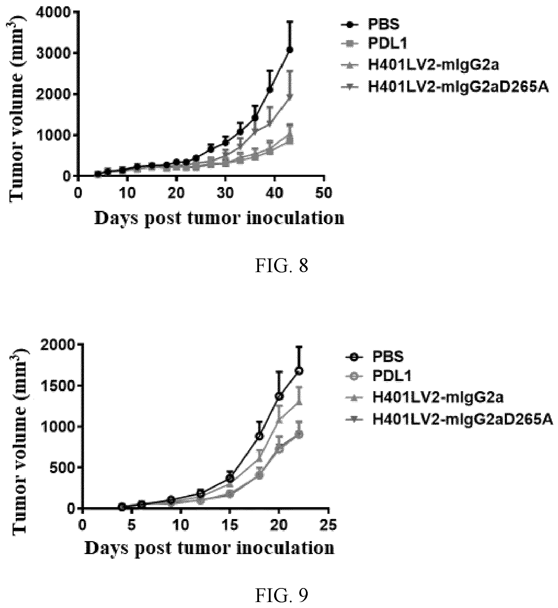

1. On day 0, hTIGIT tumor-bearing mice (purchased from Shanghai Model Organisms Center, Inc.) were injected with 2 × 10⁵ MC38 cells per mouse (FIG. 8) or 1 × 10⁶ LLC cells per mouse (FIG. 9), and the mice were randomly grouped.
2. On days 4, 7, 10, and 13, the affinity-matured antibody was intraperitoneally injected into the mice at 250 µg per mouse;
3. The tumor volume was measured every 3 days after injection of the above antibody.

As shown in FIG. 8, the results demonstrate that H401LV2-mIgG2a with ADCC effector function exhibits better efficacy in the MC38 colorectal cancer model. As shown in FIG. 9, H401LV2-mIgG2aD265A without ADCC effector function exhibits better efficacy in the LLC lung cancer model.

As can be seen from the above experimental results, the high-affinity antibody obtained in the present disclosure can bind to TIGIT. By blocking the interaction between TIGIT and CD155, the anti-tumor mechanisms of immune cells are activated.

Moreover, the terms "first" and "second" are merely intended for description but should not be construed as an indication or implication of relative importance or an implicit indication of the number of the indicated technical features. Thus, features defined by "first" or "second" may explicitly or implicitly include at least one such feature. In the description of the present disclosure, "a plurality of" means at least two, such as two or three, unless otherwise clearly and specifically limited.

In the description of the specification, reference to the term "an embodiment", "some embodiments", "an example", "a specific example" or "some examples," or the like means that a specific feature, structure, material, or characteristic described in combination with the embodiment(s) or example(s) are included in at least one embodiment or example of the present disclosure. In the specification, the schematic description of the above terms is unnecessarily against the same embodiment or example.

## Claims

1. An antibody or an antigen-binding fragment thereof capable of specifically recognizing T cell immunoreceptor with Ig and ITIM domains (TIGIT), comprising:
a heavy chain variable region with an amino acid sequence as set forth in SEQ ID NO: 7;
and
a light chain variable region with an amino acid sequence as set forth in SEQ ID NO: 8.

2. The antibody or the antigen-binding fragment thereof according to claim 1, comprising at least one of a heavy chain constant region and a light chain constant region, wherein at least a portion of the at least one of the heavy chain constant region and the light chain constant region is from at least one of a human antibody, a primate antibody, a murine antibody, or a mutant thereof.

3. The antibody or the antigen-binding fragment thereof according to claim 2, comprising the heavy chain constant region and the light chain constant region, wherein the light chain constant region and the heavy chain constant region are from a murine IgG1 antibody, IgG2a antibody, or a mutant thereof, or a human IgG1 antibody, IgG2 antibody, IgG3 antibody, IgG4 antibody, or a mutant thereof.

4. The antibody or the antigen-binding fragment thereof according to claim 1, being at least one of a single-chain antibody, a multimeric antibody, a CDR-grafted antibody, a Fab antibody, or an Fv antibody, or
the antibody is a humanized antibody.

5. A nucleic acid molecule, encoding the antibody or the antigen-binding fragment thereof according to any one of claims 1 to 4.

6. An expression vector, carrying the nucleic acid molecule according to claim 5.

7. A method for preparing the antibody or the antigen-binding fragment thereof according to any one of claims 1 to 4, comprising:
introducing the expression vector according to claim 6 into cells; and
culturing the cells under conditions suitable for protein expression and secretion to obtain the antibody or the antigen-binding fragment thereof.

8. The method of preparing the antibody or antigen-binding fragment thereof according to claim 7, wherein the cells are eukaryotic cells.

9. A composition, comprising:
the antibody or the antigen-binding fragment thereof according to any one of claims 1 to 4, the nucleic acid molecule according to claim 5, or the expression vector according to claim 6.

10. The antibody or the antigen-binding fragment thereof according to any one of claims 1 to 4, the nucleic acid molecule according to claim 5, the expression vector according to claim 6, or the composition according to claim 9, for use in the treatment or prevention of a malignant tumor.

11. The antibody or the antigen-binding fragment thereof for use according to claim 10, wherein the malignant tumor comprises at least one of lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, or head and neck cancer.

12. A medicament, comprising:
the antibody or the antigen-binding fragment thereof according to any one of claims 1 to 4, the nucleic acid molecule according to claim 5, the expression vector according to claim 6, or the composition according to claim 9,
wherein the medicament is for use in the treatment of a malignant tumor,
preferably, the malignant tumor comprises at least one of lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, or head and neck cancer.

13. Use of the antibody or the antigen-binding fragment thereof according to any one of claims 1 to 4 in the preparation of a kit for the *in vitro* detection of TIGIT.

14. A kit, comprising the antibody or the antigen-binding fragment thereof according to any one of claims 1 to 4.

## Patentansprüche

1. Antikörper oder antigenbindendes Fragment davon, der bzw. das den T-Zell-Immunrezeptor mit Ig- und ITIM-Domänen (TIGIT) spezifisch erkennen kann, umfassend:
eine variable Region der schweren Kette mit der in SEQ ID NO: 7 angegebenen Aminosäuresequenz; und
eine variable Region der leichten Kette mit der in SEQ ID NO: 8 angegebenen Aminosäuresequenz."

2. Antikörper oder antigenbindendes Fragment davon nach Anspruch 1, umfassend eine Konstantregion der schweren Kette und/oder eine Konstantregion der leichten Kette, wobei zumindest ein Teil mindestens einer der genannten Konstantregionen aus einem humanen Antikörper, einem Primatenantikörper, einem murinen Antikörper oder einer Mutante davon stammt.

3. Antikörper oder antigenbindendes Fragment davon nach Anspruch 2, umfassend die Konstantregion der schweren Kette und die Konstantregion der leichten Kette, wobei die Konstantregion der leichten Kette und die Konstantregion der schweren Kette aus einem murinen IgG1-Antikörper, einem murinen IgG2a-Antikörper oder einer Mutante davon oder aus einem humanen IgG1-Antikörper, einem humanen IgG2-Antikörper, einem humanen IgG3-Antikörper, einem humanen IgG4-Antikörper oder einer Mutante davon stammen.

4. Antikörper oder antigenbindendes Fragment davon nach Anspruch 1, wobei der Antikörper oder das antigenbindende Fragment davon mindestens eines der Folgenden ist: ein Einzelkettenantikörper, ein multimerer Antikörper, ein CDR-gepfropfter Antikörper, ein Fab-Antikörper oder ein Fv-Antikörper, oder wobei der Antikörper ein humanisierter Antikörper ist.

5. Nukleinsäuremolekül, das den Antikörper oder das antigenbindende Fragment davon nach einem der Ansprüche 1 bis 4 codiert.

6. Expressionsvektor, der das Nukleinsäuremolekül nach Anspruch 5 trägt.

7. Verfahren zur Herstellung des Antikörpers oder des antigenbindenden Fragments davon nach einem der Ansprüche 1 bis 4, umfassend:
Einbringen des Expressionsvektors nach Anspruch 6 in Zellen; und
Kultivieren der Zellen unter Bedingungen, die für die Proteinexpression und -sekretion geeignet sind, um den Antikörper oder das antigenbindende Fragment davon zu erhalten.

8. Verfahren zur Herstellung des Antikörpers oder des antigenbindenden Fragments davon nach Anspruch 7, wobei die Zellen eukaryotische Zellen sind.

9. Zusammensetzung, umfassend:
den Antikörper oder das antigenbindende Fragment davon nach einem der Ansprüche 1 bis 4,
das Nukleinsäuremolekül nach Anspruch 5 oder den Expressionsvektor nach Anspruch 6."

10. Antikörper oder antigenbindendes Fragment davon nach einem der Ansprüche 1 bis 4, Nukleinsäuremolekül nach Anspruch 5, Expressionsvektor nach Anspruch 6 oder Zusammensetzung nach Anspruch 9 zur Verwendung bei der Behandlung oder Vorbeugung eines malignen Tumors.

11. Antikörper oder antigenbindendes Fragment davon zur Verwendung nach Anspruch 10, wobei der maligne Tumor mindestens eine der folgenden Krebsarten umfasst: Lungenkrebs, Leberkrebs, Ovarialkarzinom, Zervixkarzinom, Hautkrebs, Blasenkrebs, Kolonkarzinom, Brustkrebs, Gliom, Nierenkrebs, Magenkrebs, Ösophaguskarzinom, orales Plattenepithelkarzinom oder Kopf-Hals-Krebs.

12. Arzneimittel, umfassend:
den Antikörper oder das antigenbindende Fragment davon nach einem der Ansprüche 1 bis 4,
das Nukleinsäuremolekül nach Anspruch 5, den Expressionsvektor nach Anspruch 6 oder die Zusammensetzung nach Anspruch 9,
wobei das Arzneimittel zur Verwendung bei der Behandlung eines malignen Tumors vorgesehen ist,
wobei der maligne Tumor vorzugsweise mindestens eine der folgenden Krebsarten umfasst: Lungenkrebs, Leberkrebs, Ovarialkarzinom, Zervixkarzinom, Hautkrebs, Blasenkrebs, Kolonkarzinom, Brustkrebs, Gliom, Nierenkrebs, Magenkrebs, Ösophaguskarzinom, orales Plattenepithelkarzinom oder Kopf-Hals-Krebs.

13. Verwendung des Antikörpers oder des antigenbindenden Fragments davon nach einem der Ansprüche 1 bis 4 zur Herstellung eines Kits zum In-vitro-Nachweis von TIGIT.

14. Kit, umfassend den Antikörper oder das antigenbindende Fragment davon nach einem der Ansprüche 1 bis 4.

## Revendications

1. Anticorps ou fragment de celui-ci se liant à l'antigène capable de reconnaître spécifiquement le corécepteur immunitaire des cellules T avec des domaines Ig et ITIM (TIGIT), comprenant :
une région variable de chaîne lourde avec une séquence d'acides aminés telle que définie dans SEQ ID NO: 7 ; et
une région variable de chaîne légère avec une séquence d'acides aminés telle que définie dans SEQ ID NO: 8.

2. Anticorps ou fragment de celui-ci se liant à l'antigène selon la revendication 1, comprenant au moins l'une d'une région constante de chaîne lourde et d'une région constante de chaîne légère, dans lequel au moins une partie de ladite au moins une de la région constante de chaîne lourde et de la région constante de chaîne légère provient d'au moins l'un d'un anticorps humain, d'un anticorps de primate, d'un anticorps murin, ou d'un mutant de ceux-ci.

3. Anticorps ou fragment de celui-ci se liant à l'antigène selon la revendication 2, comprenant la région constante de chaîne lourde et la région constante de chaîne légère, dans lequel la région constante de chaîne légère et la région constante de chaîne lourde proviennent d'un anticorps IgG1, d'un anticorps IgG2a murins, ou d'un mutant de ceux-ci, ou d'un anticorps IgG1, d'un anticorps IgG2, d'un anticorps IgG3, d'un anticorps IgG4 humains, ou d'un mutant de ceux-ci.

4. Anticorps ou fragment de celui-ci se liant à l'antigène selon la revendication 1, étant au moins l'un d'un anticorps à chaîne unique, d'un anticorps multimérique, d'un anticorps greffé avec des CDR, d'un anticorps Fab, ou d'un anticorps Fv, ou
l'anticorps est un anticorps humanisé.

5. Molécule d'acide nucléique, codant pour l'anticorps ou le fragment de celui-ci se liant à l'antigène selon l'une quelconque des revendications 1 à 4.

6. Vecteur d'expression, portant la molécule d'acide nucléique selon la revendication 5.

7. Méthode de préparation de l'anticorps ou du fragment de celui-ci se liant à l'antigène selon l'une quelconque des revendications 1 à 4, comprenant :
l'introduction du vecteur d'expression selon la revendication 6 dans des cellules ; et
la culture des cellules dans des conditions appropriées pour l'expression et la sécrétion de protéines afin d'obtenir l'anticorps ou le fragment de celui-ci se liant à l'antigène.

8. Méthode de préparation de l'anticorps ou du fragment de celui-ci se liant à l'antigène selon la revendication 7, dans laquelle les cellules sont des cellules eucaryotes.

9. Composition, comprenant :
l'anticorps ou le fragment de celui-ci se liant à l'antigène selon l'une quelconque des revendications 1 à 4, la molécule d'acide nucléique selon la revendication 5, ou le vecteur d'expression selon la revendication 6.

10. Anticorps ou fragment de celui-ci se liant à l'antigène selon l'une quelconque des revendications 1 à 4, molécule d'acide nucléique selon la revendication 5, vecteur d'expression selon la revendication 6, ou composition selon la revendication 9, pour une utilisation dans le traitement ou la prévention d'une tumeur maligne.

11. Anticorps ou fragment de celui-ci se liant à l'antigène pour une utilisation selon la revendication 10, dans lequel la tumeur maligne comprend au moins l'un parmi le cancer du poumon, le cancer du foie, le cancer de l'ovaire, le cancer du col de l'utérus, le cancer de la peau, le cancer de la vessie, le cancer du côlon, le cancer du sein, le gliome, le cancer du rein, le cancer de l'estomac, le cancer de l'œsophage, le carcinome épidermoïde de la cavité buccale, ou le cancer de la tête et du cou.

12. Médicament, comprenant :
l'anticorps ou le fragment de celui-ci se liant à l'antigène selon l'une quelconque des revendications 1 à 4, la molécule d'acide nucléique selon la revendication 5, le vecteur d'expression selon la revendication 6, ou la composition selon la revendication 9,
dans lequel le médicament est pour une utilisation dans le traitement d'une tumeur maligne,
de préférence, la tumeur maligne comprend au moins l'un parmi le cancer du poumon, le cancer du foie, le cancer de l'ovaire, le cancer du col de l'utérus, le cancer de la peau, le cancer de la vessie, le cancer du côlon, le cancer du sein, le gliome, le cancer du rein, le cancer de l'estomac, le cancer de l'œsophage, le carcinome épidermoïde de la cavité buccale, ou le cancer de la tête et du cou.

13. Utilisation de l'anticorps ou du fragment de celui-ci se liant à l'antigène selon l'une quelconque des revendications 1 à 4 dans la préparation d'un kit pour la détection in vitro de TIGIT.

14. Kit, comprenant l'anticorps ou le fragment de celui-ci se liant à l'antigène selon l'une quelconque des revendications 1 à 4.
